# EUROPÄISCHE PATENTANMELDUNG

(11) **EP 2 932 911 A1**
(43) Veröffentlichungstag der Anmeldung: **21.10.2015**
(21) Anmeldenummer: 15161368.4
(22) Anmeldetag: 27.03.2015
(51) Int. Cl.: A61B 17/00

(54) **Bediengerät zum Steuern einer medizinischen Einrichtung**

(30) Priorität: 17.04.2014 DE 102014105509
(71) Anmelder: Maquet GmbH, 76437 Rastatt (DE)
(72) Erfinder: Ibach, Bastian, 76185 Karlsruhe (DE); Golde, Tim, 76227 Karlsruhe (DE); Bernhart, Michael, 76137 Karlsruhe (DE)
(74) Vertreter: Schaumburg & Partner Patentanwälte (GbR)

(57) **Zusammenfassung**

Beschrieben ist ein Bediengerät (10) zum Steuern einer medizinischen Einrichtung (100), umfassend einen Gerätekörper (11) und mindestens ein an dem Gerätekörper (11) angeordnetes, von einem Benutzer durch Drücken betätigbares Schaltelement (12), über dessen Schaltzustand die medizinische Einrichtung (100) steuerbar ist, wobei durch einen in dem Gerätekörper (11) enthaltenen, mit dem Schaltelement (12) gekoppelten Funksender (20) zum Ausgeben eines den Schaltzustand angebenden Funkschaltsignals, einen separat von dem Gerätekörper (11) vorgesehenen, mit der medizinischen Einrichtung (100) koppelbarer Funkempfänger (102) zum Empfangen des von dem Funksender (20) ausgegebenen Funkschaltsignals, und eine Energieversorgungseinheit (16) zum Versorgen zumindest des Gerätekörpers (11) mit Energie, wobei der Gerätekörper (11) ein Mittel zu seiner Anbringung an einer Hand und/oder einem Arm des Benutzers umfasst.

## Beschreibung

Die Erfindung betrifft ein Bediengerät zum Steuern einer medizinischen Einrichtung, umfassend einen Gerätekörper und mindestens ein an dem Gerätekörper angeordnetes, von einem Benutzer durch Drücken betätigbares Schaltelement, über dessen Schaltzustand die medizinische Einrichtung steuerbar ist.

Während eines chirurgischen Eingriffs bedient sich ein Operateur vielfach medizinischer Einrichtungen, die ihn bei seinem Eingriff unterstützen. Beispielhaft ist hierfür ein Assistenzsystem zu nennen, das eine Nachführung eines bildgebenden Endoskops während eines laparoskopischen Eingriffs ermöglicht.

Um eine solche medizinische Einrichtung zu bedienen, muss der steril arbeitende Operateur der Einrichtung Steuerbefehle erteilen, um die gewünschten Aktionen auszulösen. Dies geschieht beispielsweise mit Hilfe eines Schaltelementes, das der Operateur mit einem Finger drückt.

Zur Durchführung des chirurgischen Eingriffs arbeitet der Operateur mit verschiedenen chirurgischen Instrumenten, die er im Laufe des Eingriffs wechselt. Um während des Eingriffs zugleich auch die medizinische Einrichtung bedienen zu können, muss dem Operateur eine Möglichkeit zur Interaktion mit der Einrichtung an die Hand gegeben werden, ohne dass der Operateur hierfür seine chirurgischen Instrumente aus der Hand legen muss und dadurch in seiner chirurgischen Tätigkeit eingeschränkt oder behindert wird.

Prinzipiell ist es denkbar, ein Schaltelement an dem chirurgischen oder laparoskopischen Element selbst zu befestigen, so dass der Operateur die medizinische Einrichtung mit einem Finger derjenigen Hand betätigen kann, die auch das Instrument hält. Beispielhaft wird hierzu auf die Druckschriften DE 10 2009 018 918 A1 und EP 1937 177 A1 verwiesen, worin Bediengeräte beschrieben sind, die sich an laparoskopischen Instrumenten befestigen lassen, um ein Assistenzsystem zur Endoskopnachführung zu steuern.

Nachteilig an diesen herkömmlichen Lösungen ist, dass für jedes einzelne Instrument ein eigenes Schaltelement vorzusehen ist. Dadurch werden diese Lösungen technisch aufwändig und teuer.

Aufgabe der Erfindung ist es, ein Bediengerät eingangs genannter Art so weiterzubilden, dass es eine technisch aufwandsarme und einfache Steuerung einer medizinischen Einrichtung durch einen Operateur ermöglicht, ohne dass der Operateur das jeweils verwendete chirurgische Instrument aus der Hand legen muss.

Die Erfindung löst diese Aufgabe durch einen in dem Gerätekörper (enthaltenen, mit dem Schaltelement gekoppelten Funksender zum Ausgeben eines den Schaltzustand angebenden Funkschaltsignals, einen separat von dem Gerätekörper vorgesehenen, mit der medizinischen Einrichtung koppelbarer Funkempfänger zum Empfangen des von dem Funksender ausgegebenen Funkschaltsignals, und eine Energieversorgungseinheit zum Versorgen zumindest des Gerätekörpers mit Energie, wobei der Gerätekörper ein Mittel zu seiner Anbringung an einer Hand und/oder einem Arm des Benutzers umfasst.

Die Erfindung sieht vor, den Gerätekörper des Bediengerätes, an dem sich das Schaltelement befindet, so auszubilden, dass der Benutzer ihn an seiner Hand, z.B. an einem Finger und/oder an einem Arm anbringen kann. Beispielsweise ist es möglich, den Gerätekörper an dem Zeigefinger derjenigen Hand anzubringen, mit der der Benutzer während eines chirurgischen Eingriffs auch die chirurgischen Instrumente hält. Indem er dann das Schaltelement auf das gerade verwendete Instrument drückt, veranlasst er das Bediengerät zur Ausgabe des Funkschaltsignals, um die Einrichtung entsprechend diesem Signal zu steuern.

Im Unterschied zu den aus dem Stand der Technik bekannten Lösungen ist es bei dem erfindungsgemäßen Bediengerät nicht mehr erforderlich, das Schaltelement von einem Instrument auf ein anderes umzumontieren. Vielmehr verbleibt das an dem Gerätekörper angeordnete Schaltelement während des gesamten Eingriffs an dem Finger bzw. der Hand des Benutzers, was die Handhabung des Bediengerätes deutlich vereinfacht. Dabei ist im Weiteren unter einem erfindungsgemäßen Schaltelement jede Art von Bedienelement zu verstehen, das sich durch Drücken betätigen lässt, wie z.B. ein Taster, der nach der Druckbetätigung in seine Ausgangslage zurückkehrt.

Indem das erfindungsgemäße Bediengerät einen in dem Gerätekörper enthaltenen Funksender und einen davon separaten, mit der medizinischen Einrichtung koppelbaren Funkempfänger aufweist, werden zusätzliche Kabelverbindungen zwischen dem Bediengerät und der medizinischen Einrichtung überflüssig. Durch eine solche drahtlose Funksteuerung (RF-Steuerung) der medizinischen Einrichtung werden mögliche Stolperfallen vermieden. Außerdem bleibt die übliche Bewegungsfreiheit des Operateurs erhalten.

Das Bediengerät weist eine Energieversorgungseinheit auf, die zumindest den Gerätekörper, d.h. die in diesem enthaltenen Funktionskomponenten mit der für den Gerätebetrieb erforderlichen Energie speist. Der separat von dem Gerätekörper vorgesehene Funkempfänger kann auch über die medizinische Einrichtung, mit der er gekoppelt ist, mit der nötigen Energie versorgt werden. Es ist jedoch ebenso möglich, dass die vorstehend genannte Energieversorgungseinheit nicht nur den Gerätekörper, sondern auch den Funkempfänger versorgt. In einer weiteren alternativen Ausführung kann der Funkempfänger auch über eine eigene Energieversorgung, z.B. eine Batterie, verfügen.

Vorzugsweise ist der Funkempfänger über eine elektrische Leitung mit der medizinischen Einrichtung verbunden. Die Ankopplung des Funkempfängers an die medizinische Einrichtung ist jedoch auch in anderer Weise, z.B. über einen Lichtleiter oder wiederum per Funk (RF) möglich.

Das Bediengerät weist vorzugsweise ein mit dem Schaltelement gekoppeltes Steuermodul auf, das den Funksender und einen Signalprozessor zum Erzeugen des von dem Funksender auszugebenden Funkschaltsignals enthält. Dieses Steuermodul kann in dem Gerätekörper selbst integriert oder als separate Baugruppe vorgesehen sein, die über elektrische Leitungen mit dem Gerätekörper verbunden wird.

In einer bevorzugten Ausführung sendet der Funksender zur erstmaligen Verbindungsaufnahme ein Paarungssignal an den Funkempfänger. Über das Paarungssignal wird der Funksender und damit der den Funksender enthaltene Gerätekörper als eine Gerätekomponente identifiziert, der es erlaubt ist, mit dem Funkempfänger und damit mit der mit dem Funkempfänger gekoppelten medizinischen Einrichtungen zu kommunizieren. Damit ist eine sichere und zuverlässige Steuerung der medizinischen Einrichtung möglich. Dies gilt insbesondere dann, wenn der den Funksender enthaltene Gerätekörper des Bediengerätes als Einwegartikel ausgebildet ist, d.h. nach einmaligem Gebrauch entsorgt und durch einen neuen Gerätekörper ersetzt wird, während der mit der medizinischen Einrichtung gekoppelte Funkempfänger über viele Eingriffe hinweg in Betrieb bleibt. Die Paarung stellt sicher, dass jeder neu zu verwendende Gerätekörper zunächst einmal daraufhin geprüft wird, ob er mit dem in der Regel wiederverwendbaren Funkempfänger kompatibel ist.

Eine besonders bevorzugte Ausgestaltung sieht eine in dem Gerätekörper angeordnete erste Funk-Sende-Empfangseinheit, die den Funksender und einen weiteren Funkempfänger enthält, und eine mit der medizinischen Einrichtung koppelbare zweite Funk-Sende-Empfangseinheit vor, die den Funkempfänger und einen weiteren Funksender enthält. In dieser Ausgestaltung kann der Gerätekörper nicht nur Funksignale aussenden, sondern auch solche empfangen.

Vorzugsweise umfasst die Energieversorgungseinheit geeignete Mittel zum Speisen des Gerätekörpers mit elektromagnetischer Energie. Diese Ausführung kann dazu genutzt werden, dass die in dem Gerätekörper enthaltenen Komponenten nach Art eines passiven RFID mit Energie gespeist werden, die der Gerätekörper etwa über eine Antenne in Form der das Funksignal bildenden elektromagnetischen Strahlung empfängt. Dabei kann die Energieversorgung über dieselbe bidirektionale Funkverbindung erfolgen, über die auch das Funkschaltsignal übertragen wird. Ebenso ist es jedoch denkbar, hierfür eine eigene Funkverbindung vorzusehen.

Die Versorgung des Gerätekörpers mit Energie kann in einer alternativen Ausführungsform auch über einen in dem Steuermodul enthaltenen Energiespeicher, z.B. eine Batterie oder einen Akkumulator, erfolgen.

In einer besonders bevorzugten Ausführungsform umfasst der Gerätekörper einen auf einen Finger des Benutzers aufsteckbaren Ring. Dabei ist der Ring vorzugsweise so ausgebildet, dass sich sein Ringdurchmesser variabel einstellen lässt. Dies lässt sich beispielsweise durch ein entsprechend verformbares Ringmaterial oder eine geeignete Einstellvorrichtung realisieren, z.B. einen Zahnriemen, eine Federanordnung oder dergleichen. Auch ist es möglich, die Innenumfangsfläche des Rings mit einer Schaumschicht zu überziehen, die den von dem Ring auf den Finger ausgeübten Druck gleichmäßig verteilt, um etwa Durchblutungsstörungen im Finger von vornherein auszuschließen.

Das Schaltelement ist vorzugsweise auf eine Außenumfangsfläche des Rings angeordnet, z.B. an einer Stelle, an der der Ring an der Unterseite der Fingerspitze anliegt. Damit lässt sich das Schaltelement besonders einfach betätigen.

In einer alternativen Ausführungsform umfasst der Gerätekörper ein flächiges, flexibles Materialstück nach Art eines medizinischen Pflasters, das eine auf die Hand und/oder den Arm des Benutzers aufklebbare Haftfläche aufweist. In dieser Ausführungsform bildet der Gerätekörper gleichsam ein "Schaltpflaster", das sich einfach und kostengünstig als Einwegartikel fertigen lässt. Auf der Haftfläche befindet sich beispielsweise ein biokompatibler Klebstoff, dessen Haftstärke zum einen so bemessen ist, dass das Schaltpflaster zuverlässig an der Zieloberfläche, etwa der Oberfläche eines Operationshandschuhs oder der Haut des Operateurs, haftet. Zum anderen soll die Haftstärke so bemessen sein, dass sich das Schaltpflaster ohne größere Beeinträchtigung, z.B. ohne Beschädigung des Operationshandschuhs, wieder von der Zieloberfläche abziehen lässt. Dabei ist die Haftfläche vorzugsweise so ausgebildet, dass sich das Schaltpflaster, nachdem es einmal von der Zieloberfläche entfernt ist, noch einmal mit hinreichender Haftstärke auf die Zieloberfläche aufkleben lässt. Die Haftfläche lässt sich in einer alternativen Ausgestaltung auch in Form eines separaten Klebebands realisieren, das beidseitig mit einem Klebstoff beschichtet ist und zum Gebrauch mit einer Seite auf den Gerätekörper und mit der anderen Seite auf die Hand des Operateurs geklebt wird.

In einer weiteren alternativen Ausführungsform umfasst der Gerätekörper einen die Hand des Benutzers zumindest teilweise umschließenden Überzug. An diesem Überzug, den sich der Benutzer über die Hand streift, befindet sich das Schaltelement. Vorzugsweise befindet sich der Überzug an der Hand, mit der der Benutzer auch das chirurgische Instrument hält. Somit kann der Benutzer das Schaltelement auf das gerade verwendete Instrument drücken, um die medizinische Einrichtung zu steuern.

Der erfindungsgemäße Überzug kann beispielsweise in Form eines Fingerhuts ausgebildet sein, der nur das letzte Fingerglied, zwei Fingerglieder oder aber auch den gesamten Finger bedeckt. Ebenso kann der Überzug derart gestaltet sein, dass er mehrere oder auch alle Finger einer Hand nach Art eines Handschuhs bedeckt.

Der Überzug besteht vorzugsweise aus einem elastischen Material, das sich der Finger- bzw. Handform anpasst, wenn der Operateur den Überzug anlegt. Hierfür kommen insbesondere Materialien in Frage, wie sie auch für Operationshandschuhe verwendet werden. Infolge seiner Elastizität passt sich der Überzug gut der individuellen Finger- bzw. Handform des Operateurs an. Es wird deshalb in aller Regel ausreichend sein, den Überzug trotz individuell unterschiedlicher Hand- und Fingergrößen in nur einer Standardgröße oder zumindest in nur wenigen Größen bereitzuhalten.

Der Überzug kann unter oder über dem sterilen OP-Handschuh getragen werden, den der Operateur während des chirurgischen Eingriffs verwendet. Aus Gründen der Ergonomie und Gebrauchstauglichkeit erscheint es jedoch vorteilhaft, den Überzug über dem OP-Handschuh zu tragen. Die Elastizität des Überzugs ermöglicht es, dass dieser faltenfrei und straff an dem Finger bzw. der Hand des Operateurs anliegt, ohne die Bewegungsfreiheit nennenswert einzuschränken.

Vorzugsweise ist das Schaltelement an einer Stelle des Überzugs angeordnet, an der sich eine Fingerspitze des Operateurs befindet. Somit ist es möglich, das Schaltelement direkt mit der Fingerspitze zu betätigen, was die Handhabung des Bediengerätes besonders einfach macht.

Das Schaltelement ist beispielsweise als Mikroschalter/-taster ausgeführt, d.h. als elektrischer Schalter/Taster, dessen Schaltkontakte im geöffneten Zustand nur einen geringen Abstand von wenigen Millimetern voneinander haben. Ein solcher Mikroschalter/-taster ist besonders für das Schalten geringer Lasten und damit für die erfindungsgemäße Erzeugung eines Schaltsignals geeignet. Durch den geringen Schaltkontaktabstand lässt sich der Mikroschalter/-taster besonders einfach betätigen. Dabei vermittelt er dem Operateur eine taktile Wahrnehmung, die dem Operateur anzeigt, wann durch sein Drücken des Schaltelementes das Schaltsignal erzeugt worden ist.

Das Schaltelement kann auch als Tastschalter mit zwei Taststufen ausgeführt sein, von denen eine erste Taststufe durch Drücken des Tastschalters bis zu einem vorbestimmten ersten Tastenhub und eine zweite Taststufe durch weiteres Drücken des Tastschalters ausgehend von der ersten Taststufe bis zu einem vorbestimmten zweiten Tastenhub betätigbar ist. Dabei ist der Tastschalter vorzugsweise so ausgebildet, dass jede Taststufe dem Operateur eine eigene taktile Wahrnehmung vermittelt, die das Erreichen der jeweiligen Taststufe angibt. Durch das Vorsehen von zwei unterscheidbaren Taststufen lassen sich zwei verschiedene Schaltsignale erzeugen, von denen eines der ersten und das andere der zweiten Taststufe zugeordnet ist. Soll dagegen nur ein einziges Schalsignal erzeugt werden, so bietet das Vorsehen von zwei Taststufen die Möglichkeit, die Erstfehlersicherheit zu erhöhen, da bei Ausfall einer der beiden Taststufen immer noch die jeweils andere Taststufe zur Erzeugung des Schaltsignals zur Verfügung steht und es somit nicht zum Ausfall des Systems führt.

In einer besonderen Ausführungsform ist der Gerätekörper einteilig ausgebildet, so dass sämtliche Komponenten des Bediengerätes mit Ausnahme des separaten Funkempfängers in dem Gerätekörper integriert sind. Dies gilt insbesondere für das mit dem Schaltelement gekoppelte Steuermodul. Alternativ ist es aber ebenso möglich, dass der Gerätekörper aus zwei separaten, über eine Leitungsstruktur elektrisch miteinander verbindbaren Teilen gebildet ist, von denen einer das Schaltelement und der andere das Steuermodul aufweist. Diese Ausführung bietet den Vorteil, dass der das Schaltelement tragende Teil des Gerätekörpers besonders klein gefertigt werden kann und somit den Operateur beim Greifen und Manipulieren der Instrumente und der zu behandelnden Gewebestrukturen nicht einschränkt.

In einer möglichen Ausgestaltung ist der das Steuermodul aufweisende Teil des Gerätekörpers als elastischer Armreif ausgebildet. Dieser kann im Bereich des Handgelenks, am Unterarm oder am Oberarm des Operateurs angebracht werden. Anstelle eines elastischen Armreifs kann auch ein Klettband verwendet werden, an welchem das Steuermodul angebracht ist.

Vorzugsweise enthält der Überzug eine Leitungsstruktur zur elektrischen Verbindung des Schaltelementes mit der Steuereinheit. Die Leitungsstruktur umfasst beispielsweise ein oder mehrere Mikrokabel. Um die in dem Überzug enthaltene Leitungsstruktur mit dem Steuermodul elektrisch zu verbinden, ist beispielsweise eine Steckverbindung vorgesehen, die aus einem oder mehreren am Ende der Leitungsstruktur angebrachten Steckern und einer bzw. mehreren an der Steuereinheit montierten Buchsen gebildet ist.

Um die Elastizität des Überzugs zu gewährleisten, ist die in dem Überzug integrierte Leitungsstruktur vorzugsweise ebenfalls elastisch ausgebildet. Dies kann beispielsweise durch eine mäanderförmige Anordnung der die Leitungsstruktur bildenden Mikrokabel erreicht werden.

In einer besonders bevorzugten Ausführungsform weist das Bediengerät eine Sensoranordnung zum Erfassen des Schaltelementes innerhalb einer vorbestimmten Aktivierungszone auf, wobei die Ausgabe des Funkschaltsignals durch das Schaltelement an die medizinische Einrichtung freigegeben ist, wenn die Sensoranordnung Schaltelement innerhalb der Aktivierungszone erfasst, und die Ausgabe des Funkschaltsignals durch das Schaltelement an die medizinische Einrichtung gesperrt ist, wenn die Sensoranordnung das Schaltelement nicht innerhalb der Aktivierungszone erfasst.

Prinzipiell ist es möglich, das erfindungsgemäße Schaltelement einfach dadurch zu betätigen, dass es auf eine beliebige Stelle gedrückt wird, die einen ausreichenden Widerstand zur Auslösung des Schaltelementes bietet. Dabei kann es jedoch leicht zu einer unbeabsichtigten Betätigung des Schaltelementes kommen, etwa wenn der Operateur versehentlich mit seinem Finger, an dem sich das Schaltelement befindet, das chirurgische Instrument oder einen anderen Gegenstand berührt. Die vorstehend genannte Sensoranordnung stellt nunmehr sicher, dass das Schaltelement nur dann zur Ausgabe des Funkschaltsignals an die medizinische Einrichtung aktiviert ist, wenn die Sensoranordnung das Schaltelement innerhalb einer vorbestimmten Aktivierungszone erfasst. Außerhalb dieser Aktivierungszone ist die Tastfunktion des Schaltelementes deaktiviert. Dabei kann die Sensoranordnung nach einem beliebigen Funktionsprinzip arbeiten, sofern sichergestellt ist, dass sie das Schaltelement innerhalb der Aktivierungszone erfasst, so dass nur dort das Schaltelement aktiviert ist.

Beispielsweise weist eine mechanisch arbeitende Sensoranordnung ein an dem Schaltelement angeordnetes erstes Formschlussteil und ein innerhalb der Aktivierungszone positionierbares zweites Formschlussteil auf, wobei das Schaltelement durch Drücken zum Ausgeben des Funkschaltsignals betätigbar ist, wenn das erste Formschlussteil und das zweite Formschlussteil ineinandergreifen. Eine solche Sensoranordnung arbeitet nach dem Schlüssel-Schloss-Prinzip, bei dem eines der beiden Formschlussteile gleichsam den Schlüssel und das andere das Schloss bildet. So kann beispielsweise das zweite Formschlussteil an dem chirurgischen Instrument angebracht werden. In diesem Fall bildet die Stelle, an der sich das zweite Formschlussteil befindet, die Aktivierungszone. Erst wenn der Operateur das Schaltelement an diese Stelle bewegt und dort die beiden Formschlussteile in Eingriff miteinander bringt, wird das Schaltelement aktiviert. Eine versehentliche Betätigung des Schaltelementes ist dadurch weitgehend ausgeschlossen.

Vorzugsweise weist das Schaltelement eine Drucktaste auf, die innerhalb eines das erste Formschlussteil bildenden Ringelementes versenkt angeordnet ist. Das zweite Formschlussteil hat in diesem Fall ein erhabenes Gegenstück, das zur Druckbeaufschlagung der Drucktaste in das Ringelement greift. In dieser Ausführungsform ist sichergestellt, dass die Drucktaste nur dann betätigt wird, wenn das das erste Formschlussteil bildende Ringelement formschlüssig auf das erhabene Gegenstück gesteckt wird, das dann an der Innenumfangsfläche des Ringelementes formschlüssig anliegt.

In einer alternativen Ausführungsform umfasst die Sensoranordnung einen Magnetschalter. Dieser ist aus einem durch ein Magnetfeld schaltbaren Element und einem das Magnetfeld erzeugenden Element gebildet. Dabei ist eines der den Magnetschalter bildenden Elemente an dem Schaltelement angeordnet, während das andere Element innerhalb der Aktivierungszone positionierbar ist. Beispielsweise ist denkbar, einen Magneten an dem medizinischen Instrument anzubringen. Bewegt dann der Operateur das an dem Überzug angeordnete, magnetisch schaltbare Element in einen den Magneten umgebenden Bereich, so wirkt das von dem Magneten verursachte Magnetfeld auf das schaltbare Element ein, wodurch dieses aktiviert wird. Wird in diesem aktivierten Zustand das Schaltelement durch Drücken betätigt, so wird das erzeugte Steuersignal an die medizinische Einrichtung gesendet. Wird dagegen das Schaltelement gedrückt, während es sich in einem Bereich befindet, in dem das Magnetfeld nicht auf das magnetisch schaltbare Element einwirkt, so ist die Übermittlung des Steuersignals an die medizinische Einrichtung gesperrt.

Das magnetisch schaltbare Element ist beispielsweise ein Reed-Schalter oder ein Hall-Sensor.

Vorzugsweise ist der Gerätekörper aus einem flüssigkeitsabweisenden Material gebildet.

Ferner betrifft die Erfindung ein Verfahren zur Bereitstellung eines Bediengerätes zum Steuern einer medizinischen Einrichtung nach dem nebengeordneten Anspruch 22.

Bei diesem Verfahren wird der Gerätekörper vorzugsweise als Einwegartikel gefertigt. In Betracht hierfür kommt insbesondere eine der oben beschriebenen Ausführungsformen wie ein Schaltpflaster oder ein Fingerring.

Die Erfindung wird im Folgenden anhand der Figuren näher erläutert. Darin zeigen:
- Fig. 1: ein erfindungsgemäßes Bediengerät zum Steuern einer medizinischen Einrichtung als Blockdiagramm,
- Fig. 2: eine schematische Darstellung des Bediengerätes nach Fig. 1 bei Verwendung eines Schaltpflasters als Gerätekörper,
- Fig. 3: eine Abwandlung des Bediengerätes nach Fig. 1 als Blockdiagramm,
- Fig. 4: eine schematische Darstellung des Bediengerätes nach Fig. 3 bei Verwendung eines Schaltpflasters als Gerätekörper,
- Fig. 5: eine schematische Darstellung, die verschiedene geeignete Positionen zeigt, in denen das Schaltpflaster an der Hand des Benutzers angebracht werden kann,
- Fig. 6: eine weitere Ausführungsform des erfindungsgemäßen Bediengerätes, bei der der Gerätekörper durch einen einteiligen, elastischen Ring gebildet ist,
- Fig. 7: eine schematische Darstellung eines mit zwei Taststufen versehenen Tastschalters, der eine beispielhafte Ausführungsform des erfindungsgemäßen Schaltelementes darstellt,
- Fig. 8: eine weitere Ausführungsform des Bediengerätes, bei welcher der zweiteilige Gerätekörper aus einem elastischen Überzug und einem Armreif gebildet ist,
- Fig. 9: das Bediengerät nach Fig. 8 in einer weiteren schematischen Darstellung,
- Fig. 10: eine Abwandlung der in Fig. 8 gezeigten Ausführungsform, bei der eine mechanische Sensoranordnung zur Anwendung kommt,
- Fig. 11: das Bediengerät nach Fig. 10 in einer weiteren schematischen Darstellung,
- Fig. 12: eine weitere Abwandlung der in Fig. 8 gezeigten Ausführungsform, bei der eine magnetische Sensoranordnung zur Anwendung kommt, und
- Fig. 13: das Bediengerät nach Fig. 12 in einer weiteren schematischen Darstellung.

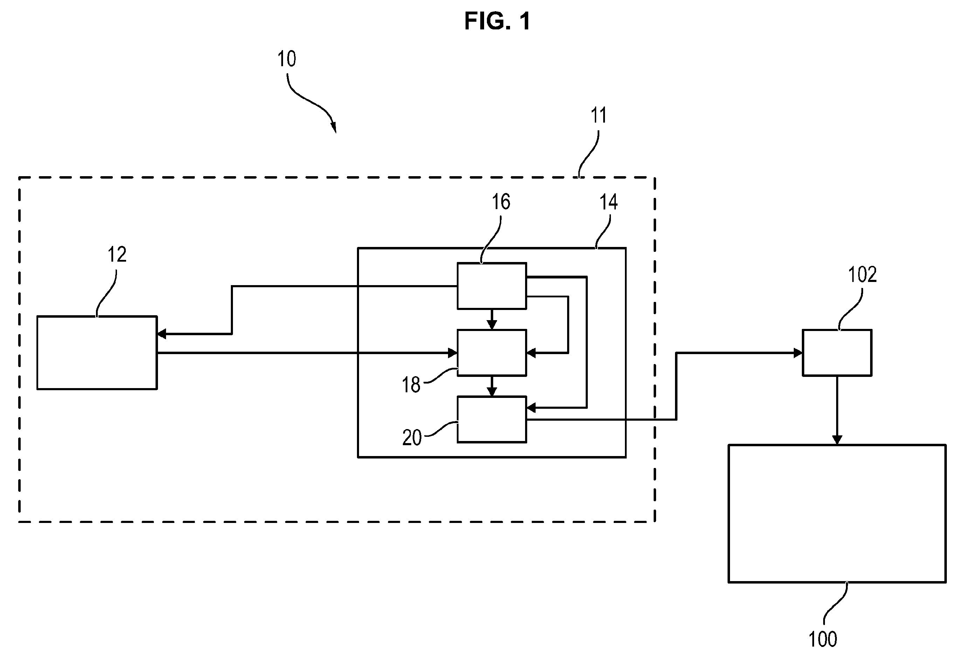

Figur 1 zeigt ein erfindungsgemäßes Bediengerät 10 in einem Blockdiagramm.

Das Bediengerät 10 weist einen Taster 12 als Schaltelement auf, den ein Operateur zum Betätigen des Bediengerätes 10 drücken kann. Das Bediengerät 10 umfasst ferner ein mit dem Taster 12 gekoppeltes Steuermodul 14, das eine Batterie 16 als Energiespeicher, einen Signalprozessor 18 und einen Funksender 20 enthält. Der Taster 12 und das Steuermodul 14 sind in der Ausführungsform nach Figur 1 in einem einteiligen Gerätekörper 11 integriert. Wie später im Detail beschrieben wird, ist es jedoch ebenso möglich, einen zweiteiligen Gerätekörper vorzusehen, wobei an dem Teil eines solchen Gerätekörpers der Taster 12 angeordnet ist, während der andere Teil des Gerätekörpers das Steuerungsmodul 14 enthält.

Das Bediengerät 10 weist ferner einen Funkempfänger 102 auf, der z.B. über eine elektrische Leitung mit einer medizinischen Einrichtung 100 gekoppelt ist. Die Einrichtung 100 ist beispielsweise ein bildgebendes Assistenzsystems, das während eines laparoskopischen Eingriffs ein mit einer Kamera ausgestattetes Endoskop nachführt. Der Funksender 102 bildet eine von dem Gerätekörper 11 separate Baugruppe.

Die in dem Steuermodul 14 enthaltene Batterie 16 speist sowohl die ebenfalls in dem Steuermodul 14 enthaltenen Komponenten, nämlich den Signalprozessor 18 und den Funksender 20, als auch den mit dem Steuermodul 14 gekoppelten Taster 12 mit elektrischer Energie. Der Signalprozessor 18 empfängt ein Betätigungssignal, das der Taster 12 an das Steuermodul 14 ausgibt, wenn der Operateur den Taster 12 durch Drücken betätigt. Dieses Betätigungssignal gibt den Schaltzustand des Tasters 12 an. Der Signalprozessor 18 setzt das von dem Taster 12 empfangene Betätigungssignal in ein Funkschaltsignal um und gibt dieses an den Funksender 20 aus. Der Funksender 20 sendet das von dem Signalprozessor 18 erzeugte Funkschaltsignal an den Funkempfänger 102, der mit der medizinischen Einrichtung 100 gekoppelt ist. In der Einrichtung 100 wird daraufhin eine dem empfangenen Funkschaltsignal entsprechende Aktion ausgelöst, z.B. eine automatische Nachführung eines auf den Operationssitus gerichteten Endoskops.

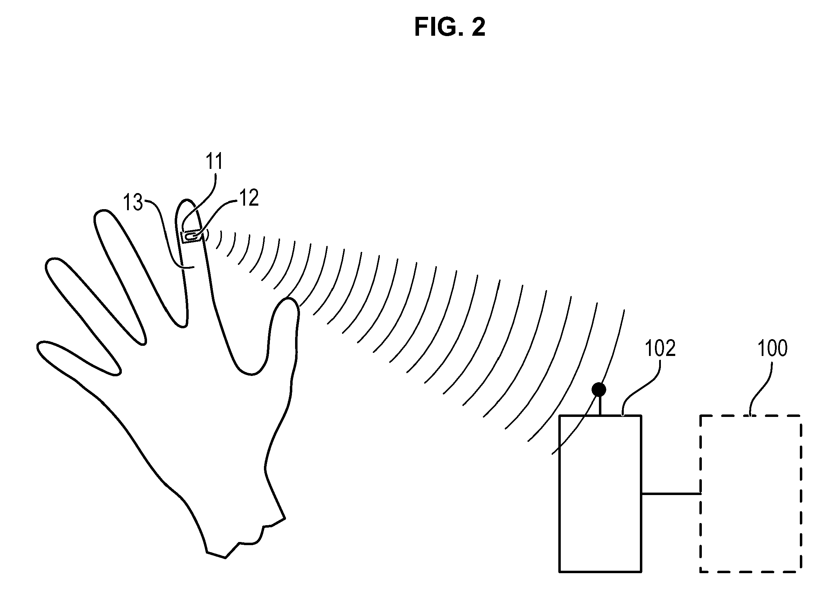

In Figur 2 ist rein schematisch eine Ausführungsform gezeigt, die nach dem in Figur 1 gezeigten Funktionsprinzip arbeitet. In der Ausführungsform nach Figur 2 ist ein einstückiger Gerätekörper 11 aus einem Schaltpflaster gebildet, d.h. aus einem flächigen, flexiblen Materialstück, das wie ein medizinisches Pflaster aufgeklebt werden kann. Dabei wird das Schaltpflaster 11 in dem vorliegenden Beispiel direkt auf die Haut eines Fingers 13 oder aber auf einen Operationshandschuh geklebt, den der Operateur während des Eingriffs trägt. In Figur 2 ist veranschaulicht, wie das Schaltpflaster 11 das Funkschaltsignal an den Funkempfänger 102 sendet. Dabei wird das Schaltpflaster 11 in der Ausführungsform nach Figur 2 durch die in Figur 1 gezeigte Batterie 16 gespeist, die in dem Schaltpflaster 11 integriert ist.

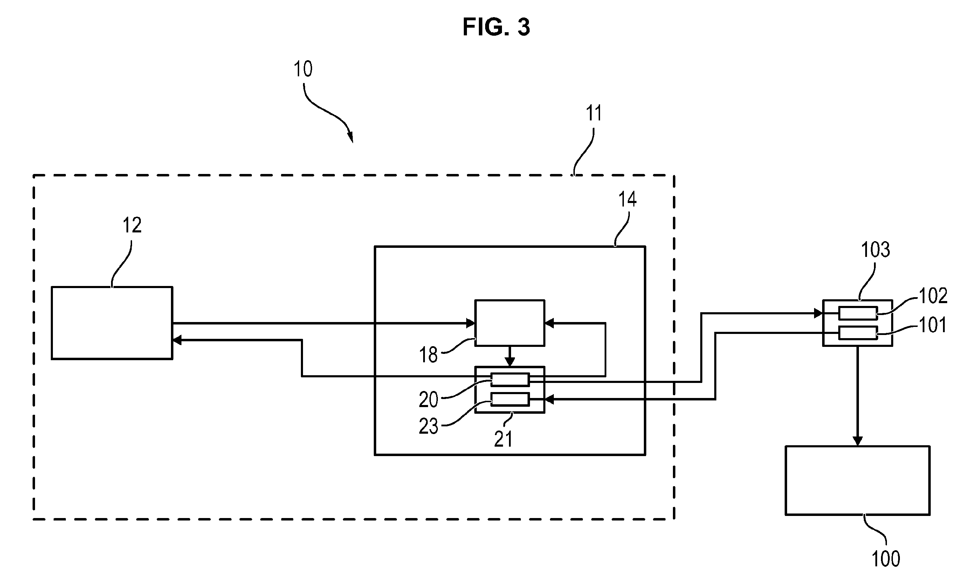

Figur 3 zeigt eine Ausführungsform des Bediengerätes 10, die gegenüber der Ausführungsform nach Figur 1 dadurch abgewandelt ist, dass der Gerätekörper 11 nicht über eine Batterie, sondern nach Art eines passiven RFID-Elementes über ein extern zugeführtes Funksignal gespeichert wird. Hierzu ist ein weiterer Funksender 101 vorgesehen, der Teil einer separaten, mit der medizinischen Einrichtung 100 gekoppelten Funk-Sende-Empfangseinheit 103 ist, die auch den Funkempfänger 102 enthält. Dementsprechend enthält das in dem Gerätekörper 11 angeordnete Steuermodul 14 eine Funk-Sende-Empfangseinheit 21, die zusätzlich zu dem Funksender 20 einen weiteren Funkempfänger 23 enthält. Um den Gerätekörper 11 mit Energie zu speisen, nimmt der Funkempfänger 23 die Energie auf, die der Funksender 101 in Form eines Funksignals aussendet. Diese aufgenommene Funkenergie wird in dem Steuermodul 14 in elektrische Energie umgesetzt.

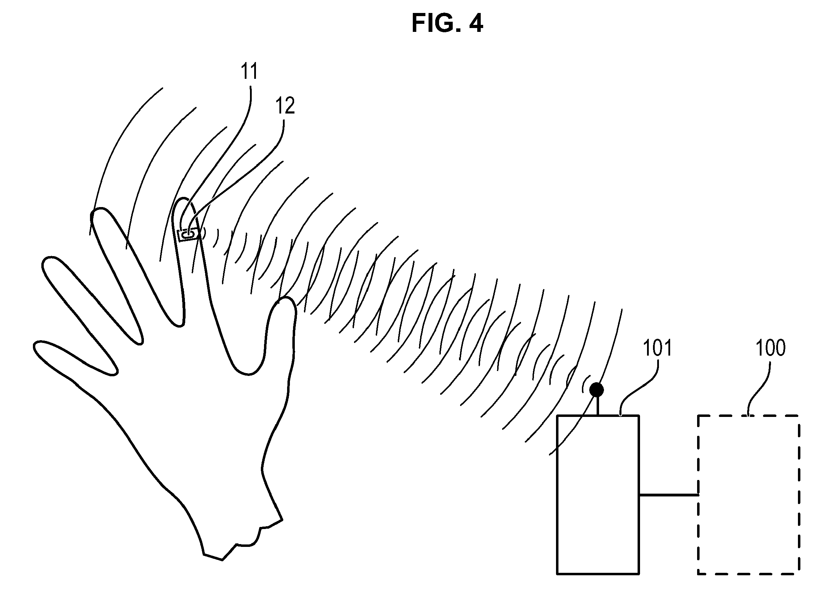

In Figur 4 ist die bidirektionale Funkkommunikation für die Ausführungsform nach Figur 3 veranschaulicht. Insbesondere zeigt Figur 4 in Ergänzung zu der in Figur 2 dargestellten Ausführungsform, wie das den Gerätekörper 11 bildende Schaltpflaster von der Funk-Sende-Empfangseinheit 101 mit Energie versorgt wird.

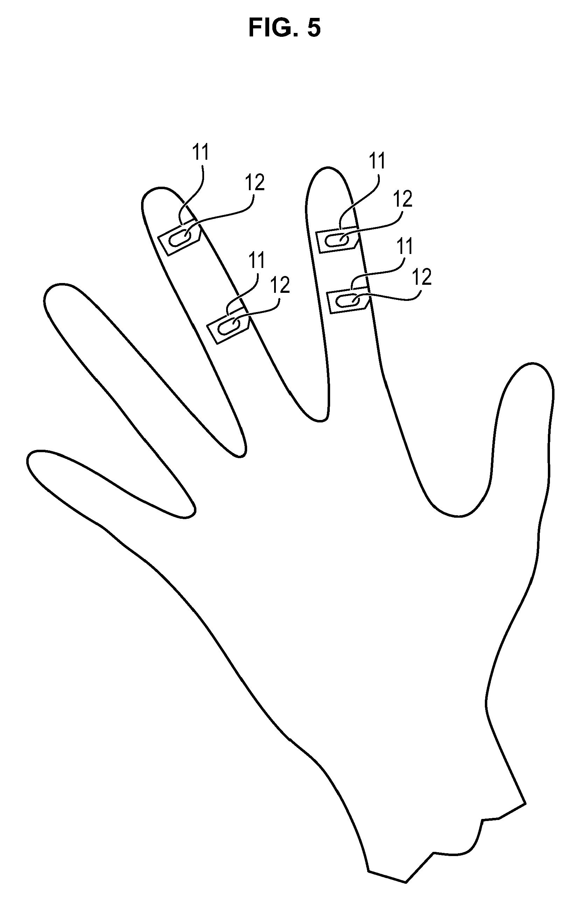

In Figur 5 ist veranschaulicht, dass das Schaltpflaster 11 je nach Bedarf an verschiedenen Stellen auf die Hand des Operateurs geklebt werden kann. In diesem Zusammenhang ist darauf hinzuweisen, dass die Erfindung nicht auf die Verwendung eines einzigen Schaltpflasters 11 (bzw. eines alternativ gestalteten Gerätekörpers 11) beschränkt ist. So ist es auch möglich, mehrere Schaltpflaster 11 an der Hand des Operateurs anzubringen.

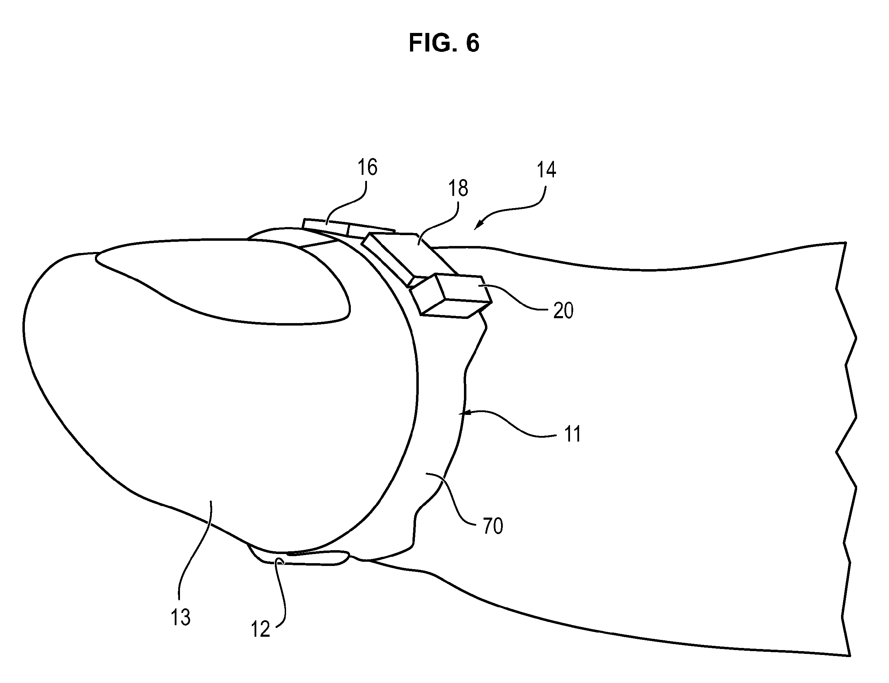

In Figur 6 ist eine Ausführungsform dargestellt, in der der Gerätekörper 11 aus einem elastischen Ring 70 gebildet ist, den der Operateur auf seinen Finger 13 aufstecken kann. Um die Fixierung des Rings 70 an dem Finger 13 zu stärken, kann auf die Innenumfangsfläche des Rings 70 ein Klebstoff aufgebracht werden. Auch in dieser Ausführungsform ist der durch den Ring 70 gebildete Gerätekörper einteilig ausgebildet. Dabei sind sowohl der Taster 12 als auch das Steuermodul 14, das die Batterie 16, den Signalprozessor 18 und den Funksender 20 enthält, auf der Außenumfangsfläche in einen Winkelabstand von etwa 180° zueinander angeordnet. Es ist jedoch ebenso möglich, dass der Ring 70 nur den einen, das Schaltelement 12 tragenden Teil eines zweiteiligen Gerätekörpers bildet. In diesem Fall wäre ein zweiter, das Steuermodul 14 tragender Teil des Gerätekörpers vorzusehen, der zum einen z.B. über eine nicht gezeigte Leitungsstruktur mit dem Ring und zum anderen per Funk mit dem Funkempfänger 102 gekoppelt ist.

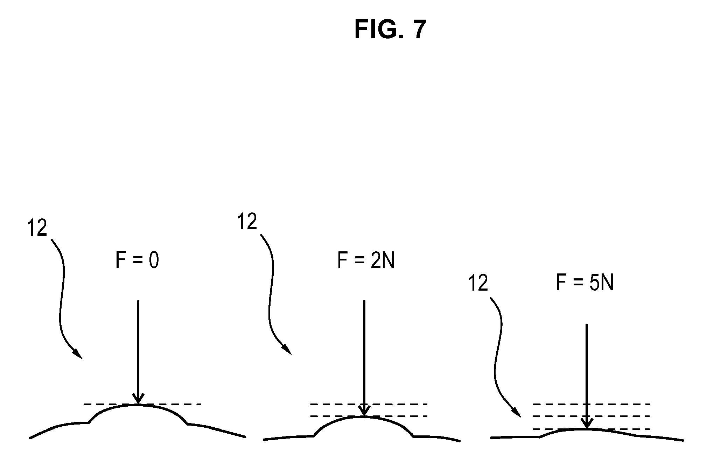

Figur 7 zeigt eine beispielhafte Ausführungsform, in der das Schaltelement 12 als ein mit zwei Taststufen versehener Tastschalter ausgebildet ist. Die beiden Taststufen werden durch unterschiedlich große Betätigungskräfte ausgelöst. Dabei wird in dem konkreten Beispiel nach Figur 7 bei einer Betätigungskraft F von 2N ein erster Tastenhub von 0,5mm erzeugt. Das Erreichen dieses Tastenhubs wird dem Operateur durch ein Klicken taktil vermittelt. Drückt dann der Operateur ausgehend von der ersten Taststufe den Tastschalter 12 mit einer Kraft F von 5N voll durch, so wird ein zweiter Tastenhub von weiteren 0,4mm und damit ein Gesamthub von 0,9mm erzeugt. Bei diesem Tastenhub ist die zweite Tastenstufe erreicht, was dem Operateur wiederum durch ein Klicken taktil vermittelt wird.

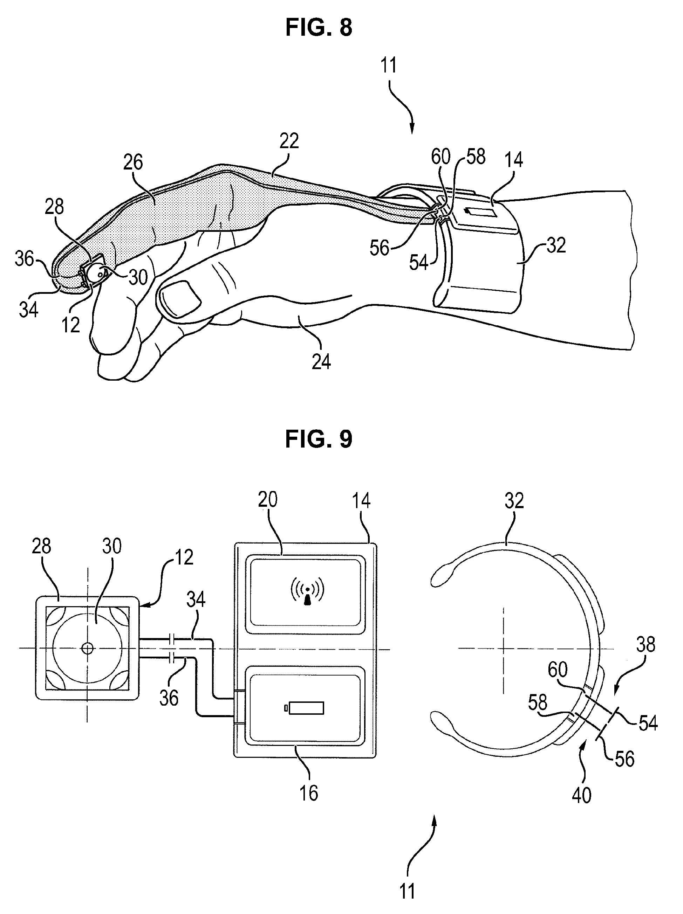

In den im Folgenden beschriebenen weiteren Ausführungsformen nach den Figuren 8 bis 13 ist der Gerätekörper 11 im Unterschied zu den vorstehenden Ausführungsformen jeweils aus zwei separaten, miteinander koppelbaren Teilen gebildet, nämlich einem elastischen Überzug 22 und einem Armreif 32.

Das Bediengerät 10 nach Figur 8 weist den elastischen Überzug 22 auf, der an die in Figur 8 mit 24 bezeichnete Hand des Operateurs derart angepasst ist, dass er sich über einen Zeigefinger 26 ziehen lässt. Der Taster 12 ist an dem Überzug 22 an einer Stelle angeordnet, an der er sich auf der Unterseite der Spitze des Zeigefingers 12 befindet, wenn der Überzug 22 auf den Zeigefinger 26 des Operateurs gezogen ist. Somit kann der Operateur mit seiner Fingerspitze den Taster 12 betätigen.

Der Taster 12 hat ein flaches, quaderförmiges Gehäuse 28, auf dem eine Drucktaste z.B. in Form Schaltmembran 30 sitzt, die durch Drücken verformbar ist. Indem die Schaltmembran 30 gedrückt wird, kommen in dem Gehäuse 28 enthaltene, in den Figuren 8 und 9 nicht explizit gezeigte Schaltkontakte miteinander in Berührung. Durch das Schließen dieser Schaltkontakte wird der Taster 12 zur Ausgabe des Schaltsignals veranlasst.

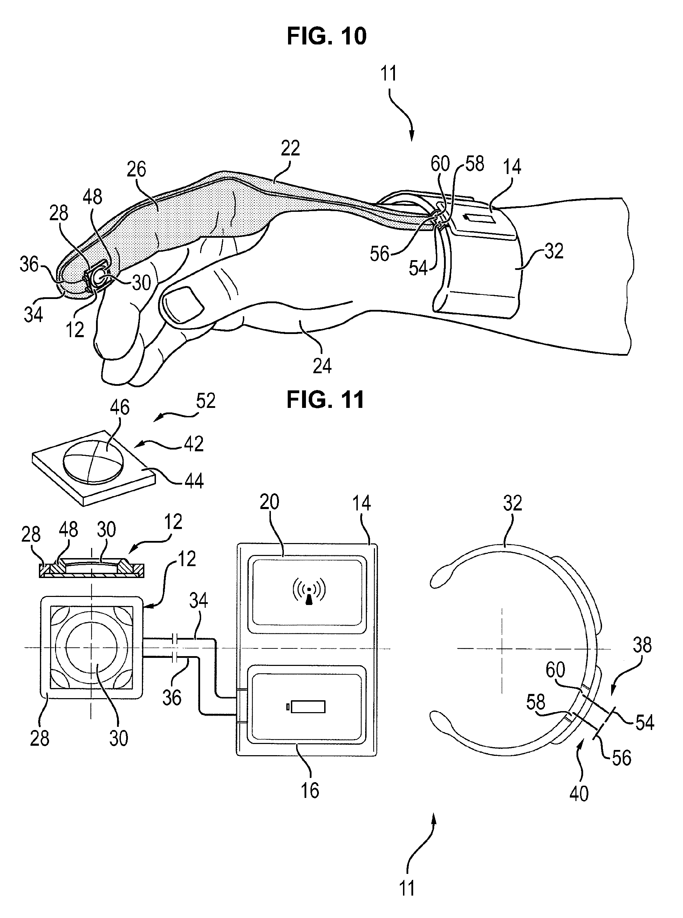

Das Bediengerät 10 nach Figur 8 weist ferner den elastischen Armreif 32 auf, den sich der Operateur auf das Handgelenk steckt. Der Armreif 32 trägt das mit dem Taster 12 elektrisch verbundene Steuermodul 14. Dabei sind der Taster 12 und das Steuermodul 14 über zwei Kabel 34 und 36 miteinander verbunden, die jeweils mit ihrem einen Ende aus dem Gehäuse 28 des Tasters 12 herausgeführt und mit ihrem anderen Ende an das Steuermodul 14 gekoppelt sind. Die Ankopplung der Kabel 34 und 36 an das Steuermodul 14 erfolgt über zwei Steckverbinder 38 und 40 (vgl. Figur 11), die jeweils aus einem an dem zugehörigen Kabel 34 bzw. 36 angeordneten Stecker 54, 56 und einer diesem Stecker 54, 56 zugeordneten Buchse 58, 60 gebildet sind, die sich an dem Steuermodul 14 befindet. Dabei ist eines der beiden Kabel 34 und 36 innerhalb des Steuermoduls 14 mit der Batterie 16 verbunden und dient damit der Speisung des Tasters 12 mit elektrischer Energie, während das andere Kabel mit dem Signalprozessor 18 gekoppelt ist. Dementsprechend sendet der Taster 12 über dieses andere der beiden Kabel 34 und 36 das Schaltsignal, das mit Betätigen der Schaltmembran 30 erzeugt wird, an den Signalprozessor 18.

In Figur 9 ist noch einmal der zweiteilige Aufbau des Gerätekörpers 11 veranschaulicht.

In den Figuren 10 und 11 ist eine weitere Ausführungsform des Bediengerätes 10 gezeigt. Diese Ausführungsform unterscheidet sich von der Ausführungsform nach Figur 8 durch eine Anordnung, die es dem Operateur ermöglicht, den Taster 12 nur bei Bedarf zur Ausgabe des Schaltsignals zu aktivieren.

Diese Anordnung beinhaltet ein Formschlussteil 42, das mit dem Taster 12 in nachfolgend erläuterter Weise zusammenwirkt, um diesen zur Ausgabe des Schaltsignals zu aktivieren. Das Formschlussteil 42 umfasst ein plattenartiges Element 44, auf dessen Oberseite ein erhabenes, kreisrundes Gegenstück 46 sitzt. Das Gegenstück 46 ist in Form und Größe so an die Schaltmembran 30 des Tasters 12 angepasst, dass es auf die Schaltmembran 30 drückt, wenn der Operateur das Tastelement 12 auf das Formschlussteil 42 aufsetzt. Um ein formschlüssiges Aufsetzen des Tasters 12 auf dem Formschlussteil 42 zu ermöglichen, ist an dem Taster 12 ein zu dem Formschlussteil 42 korrespondierendes Formschlussteil 48 in Form eines kreisrunden, die Schaltmembran 30 konzentrisch umgebenden Rings 48 ausgebildet. Drückt der Operateur die Taste 12 passgenau auf das Formschlussteil 42, so setzt der Ring 48 so auf die Oberseite des Formschlussteils 42 auf, dass er am Umfang des erhabenen Gegenstücks 46 anliegt. In dieser formschlüssigen Anlage drückt dann das erhabene Gegenstück 46 auf die Schaltmembran 30, wodurch die in dem Gehäuse 28 des Tasters 12 enthaltenen Schaltkontakte geschlossen werden.

In Figur 11 ist der mit dem Ring 48 versehene Taster 12 auch im Querschnitt gezeigt. Dieser Querschnittsansicht ist zu entnehmen, dass die Schaltmembran 30 innerhalb des Rings 48 versenkt angeordnet ist. Diese versenkte Anordnung bedeutet, dass der Ring 48 im Querschnitt über die Schaltmembran 30 hinaussteht, so dass die Schaltmembran 30 nicht betätigt wird, wenn der Taster 12 und damit der überstehende Ring 48 beispielsweise auf eine plane oder nur leicht gewölbte Fläche aufsetzt. Allein wenn der Taster 12 passgenau auf das Formschlussteil 42 aufsetzt, ermöglicht das in den Ring 48 greifende Gegenstück 46 eine Betätigung der Schaltmembran 30.

Die beiden Formschlussteile 42 und 48 bilden somit eine in Figur 10 allgemein mit 52 bezeichnete Sensoranordnung, die es ermöglicht, den Taster 12 in einer vorbestimmten Aktivierungszone zu erfassen und eine Ausgabe des Schaltsignals an das Steuermodul 14 nur dann freizugeben, wenn sich der Taster 12 in der Aktivierungszone befindet. Die Aktivierungszone ist dabei durch die Positionierung des Formschlussteils 42 definiert, das beispielsweise auf einem chirurgischen Instrument angebracht ist.

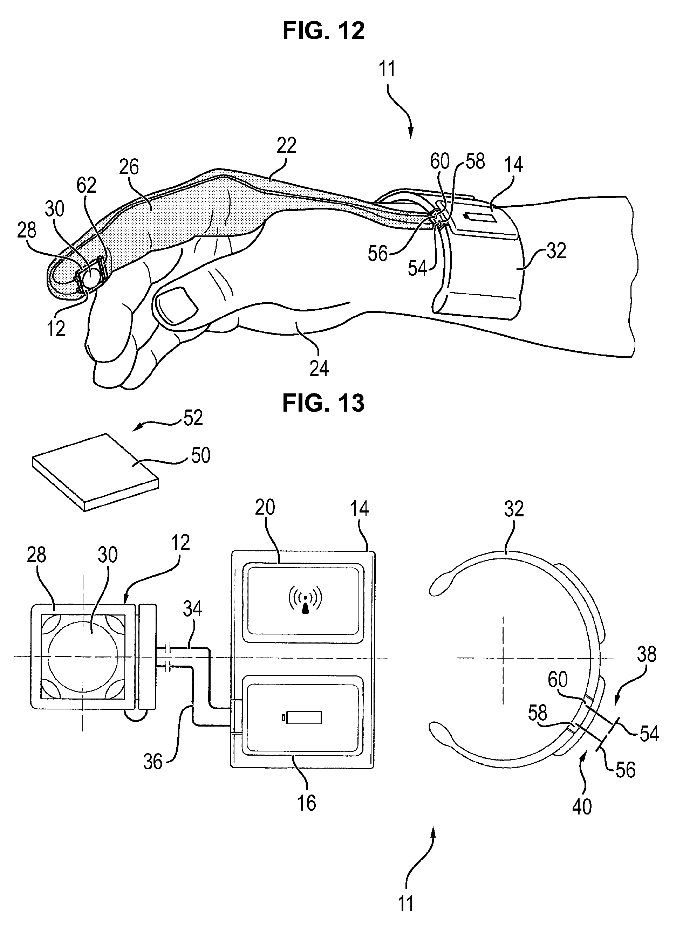

In den Figuren 12 und 13 ist eine weitere Ausführungsform gezeigt, die sich von der Ausführungsform nach den Figuren 10 und 11 durch eine andere Art der Sensoranordnung 52 unterscheidet.

Während in der Ausführungsform nach den Figuren 10 und 11 die beiden Formschlussteile 42 und 48 gleichsam eine mechanisch arbeitende Sensoranordnung zur Erfassung eines Freigabezustands des Tastelementes 12 bilden, ist in dieser Ausführungsform die Sensoranordnung eine elektromagnetisch arbeitende Anordnung.

Die in den Figuren 12 und 13 gezeigte Sensoranordnung 52 ist aus einem Magnetschalter gebildet, der ein neben dem Gehäuse 28 des Tasters 12 angeordnetes, magnetisch schaltbares Element in Form eines Reed-Schalters 62 und eine mit dem Reed-Schalter 62 magnetisch wechselwirkende Magnetplatte 50 umfasst. Die Magnetplatte 50 erzeugt ein Magnetfeld, das genutzt werden kann, um eine Aktivierungszone zu definieren, innerhalb der der Taster 12 zur Ausgabe des Schaltsignals aktiviert wird. Die Aktivierungszone ist wiederum durch die Positionierung der Magnetplatte 50 definiert, wobei die Magnetplatte 50 vorzugsweise an dem chirurgischen Instrument angeracht wird. Bewegt nämlich der Operateur den an seinem Zeigefinger 26 gehaltenen Taster 12 in das von der Magnetplatte 50 verursachte Magnetfeld, so wirkt Letzteres auf den an dem Taster 12 angeordneten Reed-Schalter 62 so ein, dass dieser geschlossen und damit der Taster 12 über ein entsprechendes Signal aktiviert wird. Drückt dann der Operateur im aktivierten Zustand den Taster 12 mit seiner Schaltmembran 30 auf die Magnetplatte 50, so sendet der Taster 12 das Schaltsignal über den in dem Steuermodul 14 enthaltene Funksender 20 an die medizinische Einrichtung 100.

Wird dagegen der Taster 12 außerhalb der Aktivierungszone, d.h. außerhalb des Wirkungsbereichs des von der Magnetplatte 50 erzeugten Magnetfeldes betätigt, so ist die Ausgabe des Schaltsignals durch den in dem Taster 12 enthaltenen Reed-Schalter 62 gesperrt. Demzufolge bewirkt eine Betätigung der Schaltmembran 30 des Tasters 12 in diesem Zustand keine Ausgabe des Schaltsignals an die medizinische Einrichtung 100.

Die vorstehend erläuterten Ausführungsformen der Sensoranordnung sind lediglich beispielhaft zu verstehen. So kann etwa der Reed-Schalter 62 durch ein mechanisch schaltbares Element anderer Art, z.B. einen Hall-Sensor, ersetzt werden.

Überhaupt sind auch andere Sensoranordnungen zur Erfassung des Tasters 12 in der Aktivierungszone als die mechanische Anordnung nach der in den Figuren 10 und 11 gezeigten Ausführungsform oder die elektromagnetische Anordnung nach der in den Figuren 12 und 13 gezeigten Ausführungsform verwendbar, z.B. eine mit optischen Mitteln wie einer Leuchtdiode und einer Fotodiode arbeitende Anordnung. Alternativ ist auch eine Sensoranordnung denkbar, bei der die Aktivierungszone durch eine chemische Substanz gekennzeichnet und über einen entsprechenden chemischen Sensor erfasst wird, der am Taster 12 angeordnet ist.

Die vorstehend beschriebenen Ausführungsformen dienen lediglich der beispielhaften Veranschaulichung des Erfindungsgegenstandes. Insbesondere lassen sich die für die einzelnen Ausführungsformen beschriebenen Teilaspekte in verständiger Weise miteinander kombinieren. Dies gilt insbesondere für die konkrete Ausgestaltung des ein- oder mehrteiligen Gerätekörpers 11 einerseits und die konkrete Ausgestaltung des Schaltelementes 12 andererseits. Gleiches gilt für die Energieversorgung des Gerätekörpers 11. So kann beispielsweise das in den Figuren 8 bis 13 gezeigte Steuermodul 14 auch extern nach Art eines passiven RFID-Elementes mit Energie versorgt werden.

## Patentansprüche

1. Bediengerät (10) zum Steuern einer medizinischen Einrichtung (100), umfassend einen Gerätekörper (11) und mindestens ein an dem Gerätekörper (11) angeordnetes, von einem Benutzer durch Drücken betätigbares Schaltelement (12), über dessen Schaltzustand die medizinische Einrichtung (100) steuerbar ist,
**gekennzeichnet durch** einen in dem Gerätekörper (11) enthaltenen, mit dem Schaltelement (12) gekoppelten Funksender (20) zum Ausgeben eines den Schaltzustand angebenden Funkschaltsignals,
einen separat von dem Gerätekörper (11) vorgesehenen, mit der medizinischen Einrichtung (100) koppelbarer Funkempfänger (102) zum Empfangen des von dem Funksender (20) ausgegebenen Funkschaltsignals, und
eine Energieversorgungseinheit (16) zum Versorgen zumindest des Gerätekörpers (11) mit Energie, wobei
der Gerätekörper (11) ein Mittel zu seiner Anbringung an einer Hand und/oder einem Arm des Benutzers umfasst.

2. Bediengerät (10) nach Anspruch 1, **gekennzeichnet durch** ein mit dem Schaltelement (12) gekoppeltes Steuermodul (14), das den Funksender (20) und einen Signalprozessor (18) zum Erzeugen des von dem Funksender (20) auszugebenden Funkschaltsignals enthält.

3. Bediengerät (10) nach einem der vorhergehenden Ansprüche, **dadurch gekennzeichnet, dass** der Gerätekörper (11) einen auf einen Finger des Benutzers aufsteckbarer Ring (70) umfasst.

4. Bediengerät (10) nach Anspruch 3, **dadurch gekennzeichnet dass** das Schaltelement (12) auf einer Außenumfangsfläche des Rings (70) angeordnet ist.

5. Bediengerät (10) nach Anspruch 1 oder 2, **dadurch gekennzeichnet, dass** der Gerätekörper (11) ein flächiges, flexibles Materialstück nach Art eines medizinischen Pflasters umfasst, das eine auf die Hand und/oder den Arm des Benutzers aufklebbare Haftfläche aufweist.

6. Bediengerät (10) nach Anspruch 1 oder 2, **dadurch gekennzeichnet, dass** der Gerätekörper (11) einen die Hand des Benutzers zumindest teilweise umschließenden Überzug (26) umfasst.

7. Bediengerät (10) nach einem der vorhergehenden Ansprüche, **dadurch gekennzeichnet, dass** das Schaltelement (12) ein Tastschalter mit zwei Taststufen ist, von denen eine erste Taststufe durch Drücken des Tastschalters bis zu einem vorbestimmten ersten Tastenhub und eine zweite Taststufe durch weiteres Drücken des Tastschalters ausgehend von der ersten Taststufe bis zu einem vorbestimmten zweiten Tastenhub betätigbar ist.

8. Bediengerät (10) nach einem der vorhergehenden Ansprüche, **dadurch gekennzeichnet, dass** der Gerätekörper (11) einteilig ausgebildet ist.

9. Bediengerät (10) nach einem der Ansprüche 1 bis 7, **dadurch gekennzeichnet, dass** der Gerätekörper (11) aus zwei separaten, vorzugsweise über eine Leitungsstruktur (34, 36) elektrisch miteinander verbindbaren Teilen (22, 32) gebildet ist, von denen das mindestens eine Schaltelement (12) und der andere das Steuermodul (14) aufweist.

10. Bediengerät (10) nach Anspruch 9, **dadurch gekennzeichnet, dass** der das Steuermodul (14) aufweisende Teil des Gerätekörpers (11) ein elastischer Armreif (32) ist.

11. Bediengerät (10) nach einem der vorhergehenden Ansprüche, **gekennzeichnet durch** eine Sensoranordnung (52) zum Erfassen des Schaltelementes (12) innerhalb einer vorbestimmten Aktivierungszone, wobei die Ausgabe des Funkschaltsignals **durch** den Funksender (20) freigegeben ist, wenn die Sensoranordnung (52) das Schaltelement (12) innerhalb der Aktivierungszone erfasst, und die Ausgabe des Funkschaltsignals gesperrt ist, wenn die Sensoranordnung (52) das Schaltelement (12) nicht innerhalb der Aktivierungszone erfasst.

12. Bediengerät (10) nach Anspruch 11, **dadurch gekennzeichnet, dass**
die Sensoranordnung ein an dem Schaltelement (12) angeordnetes erstes Formschlussteil (48) und ein innerhalb der Aktivierungszone positionierbares zweites Formschlussteil (42) aufweist; und
das Schaltelement (12) durch Drücken zum Ausgeben des Funkschaltsignals betätigbar ist, wenn das erste Formschlussteil (48) und das zweite Formschlussteil (42) ineinander greifen.

13. Bediengerät (10) nach Anspruch 12, **dadurch gekennzeichnet, dass** das Schaltelement (12) eine Drucktaste (30) aufweist, die innerhalb des ersten Formschlussteils (48) versenkt angeordnet ist; und
das zweite Formschlussteil (42) ein erhabenes Gegenstück (46) aufweist, das zur Druckbeaufschlagung der Drucktaste (30) in das erste Formschlussteil (48) greift.

14. Bediengerät (10) nach Anspruch 11, **dadurch gekennzeichnet, dass** die Sensoranordnung (52) einen Magnetschalter umfasst, der aus einem durch ein Magnetfeld schaltbaren Element und einem das Magnetfeld erzeugenden Element (50) gebildet ist, wobei eines der Elemente des Magnetschalters an dem Schaltelement (12) angeordnet ist und das andere Element innerhalb der Aktivierungszone positionierbar ist.

15. Verfahren zur Bereitstellung eines Bediengerätes (10) zum Steuern einer medizinischen Einrichtung (100), mit folgenden Schritten:
Herstellen eines für das Bediengerät (10) bestimmten Gerätekörpers (11), der mindestens ein von einem Benutzer von Hand durch Drücken betätigbares Schaltelement (12), über dessen Schaltzustand die medizinische Einrichtung (100) steuerbar ist, einen mit dem Schaltelement (12) gekoppelten Funksender (20) zum Ausgeben eines den Schaltzustand angebenden Funkschaltsignals und ein Mittel zur Anbringung des Gerätekörpers (11) an einer Hand und/oder einem Arm des Benutzers aufweist;
Sterilisieren des hergestellten Gerätekörpers (11); und
Anordnen des sterilisierten Gerätekörpers (11) in einer Verpackung, die von dem Benutzer zum Gebrauch des Bediengerätes (10) zu öffnen ist.
